# EUROPEAN PATENT SPECIFICATION

(11) **EP 3 101 002 B1**
(45) Date of publication and mention of the grant of the patent: **15.08.2018**
(21) Application number: 15740380.9
(22) Date of filing: 22.01.2015
(51) Int. Cl.: C07C 309/43, C07C 303/22, C09K 11/06, H01L 51/50, H01L 51/00

(54) **ARYL SULFONIC ACID COMPOUND AND USE OF SAME**
ARYL-SULFONSÄURE-VERBINDUNG UND VERWENDUNG DAVON
COMPOSÉ D'ACIDE ARYLSULFONIQUE ET SON UTILISATION

(30) Priority: 27.01.2014 JP 2014012672
(43) Date of publication of application: 07.12.2016
(73) Proprietor: Nissan Chemical Industries, Ltd., Tokyo 101-0054 (JP)
(72) Inventor: NAKAIE, Naoki, Funabashi-shi Chiba 274-0052 (JP)
(74) Representative: Bailey, Sam Rogerson
(86) International application number: PCT/JP2015/051672
(87) International publication number: WO 2015/111654

(56) References cited:
- EP-A1- 0 354 329
- EP-A1- 2 246 325
- WO-A1-2009/096352
- JP-A- H0 267 262

## Description

### TECHNICAL FIELD

This invention relates to an arylsulfonic acid compound and the use thereof.

### BACKGROUND ART

Organic electroluminescent (EL) devices are expected to see practical application in such fields as displays and lighting. A variety of research is being carried out on materials and device structures with the aim of achieving such properties as low-voltage driving, high brightness and longevity.

A plurality of functional thin-films are used in organic EL devices, one of which, the hole injection layer, is responsible for transferring charge between an anode and a hole-transporting layer or an emissive layer, and thus serves an important function in achieving low-voltage driving and high brightness in organic EL devices.

Processes for forming the hole injection layer are broadly divided into dry processes such as vapor deposition and wet processes such as spin coating. On comparing these processes, wet processes are better able to efficiently produce thin-films having a high flatness over a large surface area and therefore are often used particularly in the field of displays.

In view of the above, improvements in the wet process materials for the hole injection layer are constantly being sought. In particular, to help improve the brightness and longevity characteristics of organic EL devices, there has been a growing demand for materials that provide a thin-film having excellent flatness and charge transportability.

### PRIOR ART DOCUMENT

### PATENT DOCUMENT

Patent Document 1: WO 2009/096352

### SUMMARY OF THE INVENTION

### PROBLEMS TO BE SOLVED BY THE INVENTION

It is therefore an object of this invention to provide varnish materials that reproducibly give charge-transporting thin-films having a high flatness.

### MEANS FOR SOLVING THE PROBLEMS

The inventor, after conducting extensive investigations, has discovered that arylsulfonic acid compounds having a structure in which a sulfonic acid group-substituted naphthyl group and a 2,3,4,5-tetrafluoro-4-nitrophenyl group are linked through an ether group not only exhibit non-crystallinity at room temperature, they also have an excellent solubility in various types of organic solvents and moreover are capable of functioning as dopants; that by dissolving such an arylsulfonic acid compound together with a charge-transporting substance in a solvent, there can be obtained a varnish capable of maintaining a suitable liquid film state during baking, which is essential for achieving a high flatness in a charge-transporting thin-film; and that a thin-film obtained from this charge-transporting varnish can be suitably used as a hole injection layer in organic EL devices.

Accordingly, the invention provides the following arylsulfonic acid compound.
1. An arylsulfonic acid compound of formula (1)
   [Chemical Formula 1]

   **Ar¹-O-Ar²** **(1)**

   (wherein Ar¹ is a group of formula (2), and Ar² is a group of formula (3) 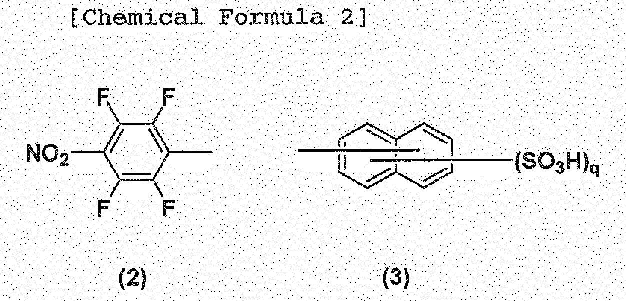 (q being an integer from 1 to 4)).
2. The arylsulfonic acid compound of 1 above which has any one of formulas (1-1) to (1-4) 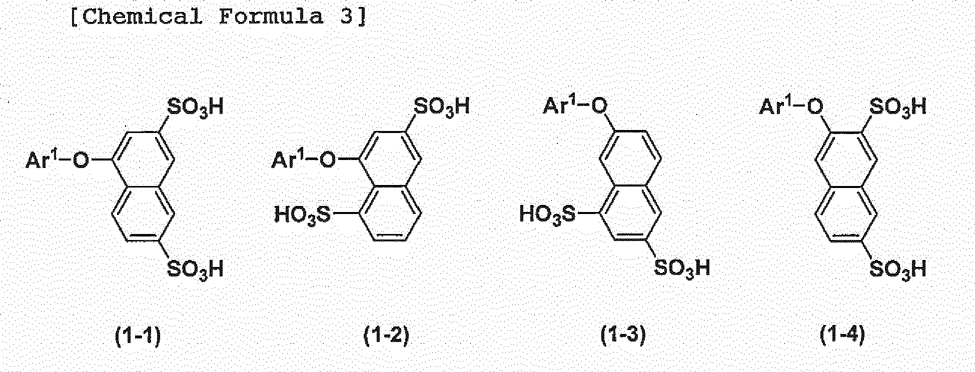 (wherein Ar¹ is as defined above).
3. A dopant consisting of the arylsulfonic acid compound of 1 or 2 above.
4. A charge-transporting material comprising the dopant of 3 above and a charge-transporting substance.
5. A charge-transporting varnish comprising the dopant of 3 above, a charge-transporting substance and an organic solvent.
6. A charge-transporting thin-film obtained using the charge-transporting varnish of 5 above.
7. An organic EL device comprising the charge-transporting thin-film of 6 above.
8. A method for producing a charge-transporting thin-film, which method comprises using the charge-transporting varnish of 5 above.
9. A method of preparing the arylsulfonic acid compound of 1 above, which method is characterized by comprising the steps of: reacting 2,3,4,5,6-pentafluoronitrobenzene with a naphthalenesulfonic acid salt of formula (5) to form an arylsulfonic acid salt of formula (1') 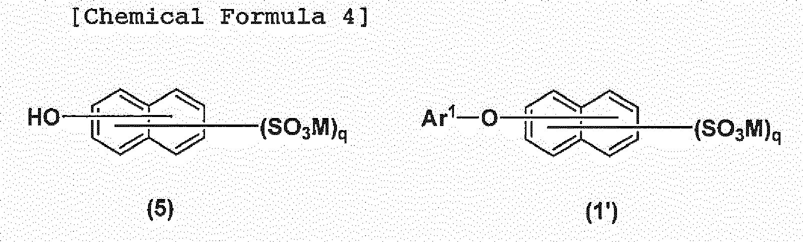 (wherein M is an alkali metal atom, and q and Ar¹ are as defined above); and ion-exchange treating the salt.

It should be noted that Patent Document 1 discloses an arylsulfonic acid having a structure in which a sulfonic acid group-substituted naphthyl group and a given aryl group are linked through an ether group. However, the arylsulfonic acid compound of the present invention is not specifically disclosed in Patent Document 1. Nor is there any language in Patent Document 1 which teaches or suggests that, by dissolving the arylsulfonic acid compound of the invention as a dopant, together with a charge-transporting substance, in an organic solvent, there can be obtained a varnish capable of maintaining the suitable liquid film state during baking that is required for achieving the high flatness of a charge transporting thin-film.

### ADVANTAGEOUS EFFECTS OF THE INVENTION

The arylsulfonic acid compound of the invention not only exhibits non-crystallinity at room temperature, it has excellent solubility in various organic solvents and moreover is capable of functioning as a dopant. Also, the arylsulfonic acid compound-containing varnish of the invention, because agglomeration of the liquid film when the varnish has been applied onto a substrate (i.e., the applied film) is suppressed, is able to maintain the liquid film state required to achieve a high flatness in a thin-film.

Therefore, a varnish capable of reproducibly giving charge-transporting thin-films of high flatness can be prepared by dissolving the arylsulfonic acid compound of the invention, together with, for example, a charge-transporting substance made of an aniline derivative, in a solvent. Because a thin-film obtained from such a charge-transporting varnish has excellent flatness and charge transportability, it can be suitably used as a hole injection layer for an organic EL device.

Moreover, the arylsulfonic acid compound of the invention can function as a dopant, and the thin-film formed when the compound is used together with a charge-transporting substance exhibits high charge transportability. Hence, the use of such a thin-film as, for example, a capacitor electrode-protecting film, an antistatic film, or an anode buffer layer in an organic thin-film solar cell is also promising.

### BRIEF DESCRIPTION OF THE DIAGRAMS

[FIG. 1] FIG. 1 shows a micrograph of the surface of the thin-film obtained in Working Example 3-1.
[FIG. 2] FIG. 2 shows a micrograph of the surface of the thin-film obtained in Comparative Example 3-1.

### EMBODIMENT FOR CARRYING OUT THE INVENTION

The arylsulfonic acid compound of the invention has formula (1) below.
[Chemical Formula 5]

**Ar¹-O-Ar²** **(1)**

Ar¹ is a group of formula (2) below, and Ar² is a group of formula (3) below.

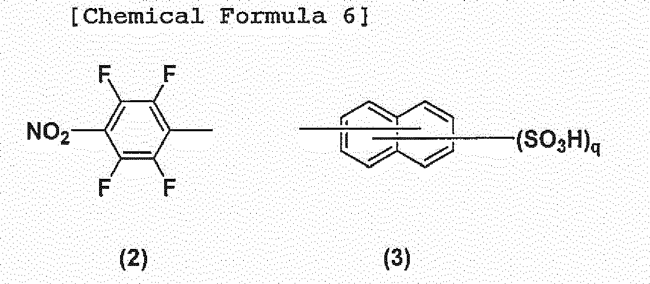

Here, the letter q, which represents the number of sulfonic acid groups substituted on the naphthyl group, is an integer from 1 to 4. Taking into account the balance between the solubility of the arylsulfonic acid compound and the -availability of the starting compounds, q is most preferably 2.

The arylsulfonic acid compound of the invention can be obtained by reacting 2,3,4,5,6-pentafluoronitrobenzene (formula (4) below) with the naphthalenesulfonic acid salt of formula (5) below to form an arylsulfonic acid salt of formula (1') below, and subjecting the resulting salt to ion-exchange treatment. 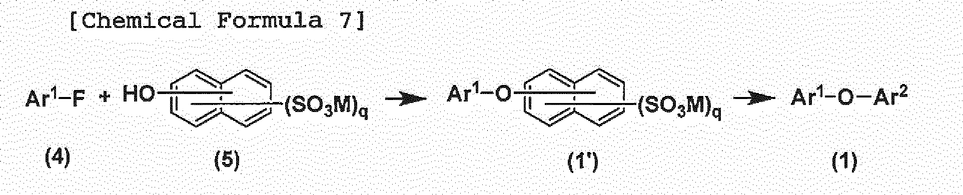 In these formulas, M is an alkali metal atom such as sodium or potassium, and q, Ar¹ and Ar² are as defined above.

Examples of the naphthalenesulfonic acid salt of formula (5) include, but are not limited to, 1-hydroxynaphthalene-3,6-disulfonic acid disodium salt, 1-hydroxynaphthalene-3,8-disulfonic acid disodium salt, 2-hydroxynaphthalene-6,8-disulfonic acid disodium salt, 3-hydroxynaphthalene-2,7-disulfonic acid disodium salt, 1-hydroxynaphthalene-3,6-disulfonic acid dipotassium salt, 1-hydroxynaphthalene-3,8-disulfonic acid dipotassium salt, 2-hydroxynaphthalene-6,8-disulfonic acid dipotassium salt and 3-hydroxynaphthalene-2,7-disulfonic acid dipotassium salt. The napthalenesulfonate may be a hydrate.

The charging ratio between 2,3,4,5,6-pentafluoronitrobenzene and the naphthalenesulfonic acid salt of formula (5) may be set to about 0.5 to 2.0 moles of the naphthalenesulfonic acid salt per mole of 2,3,4,5,6-pentafluoronitrobenzene. From the standpoint of efficiently obtaining the arylsulfonic acid salt of formula (1'), this is preferably about 0.8 to 1.2 moles.

From the standpoint of efficiently obtaining the arylsulfonic acid salt of formula (1'), it is preferable to use a base in the above reaction.

Illustrative examples include, without particular limitation so long as use in this type of reaction is possible: uncombined alkali metals, alkali metal hydrides, alkali metal hydroxides, alkoxy alkali metals, alkali metal carbonates and alkali metal bicarbonates such as lithium, sodium, potassium, lithium hydride, sodium hydride, lithium hydroxide, potassium hydroxide, t-butoxylithium, t-butoxysodium, t-butoxypotassium, sodium hydroxide, potassium hydroxide, sodium carbonate, potassium carbonate, sodium bicarbonate and potassium bicarbonate; alkaline earth metal carbonates such as calcium carbonate; organolithium compounds such as n-butyllithium, s-butyllithium and t-butyllithium; and amines such as triethylamine, diisopropylethylamine, tetramethylethylenediamine, triethylenediamine and pyridine. From the standpoint of ease of handling, sodium hydride, sodium carbonate and potassium carbonate are especially preferred.

It is generally sufficient for the amount of base used to be about 1.0 to 1.5 equivalents relative to the naphthalenesulfonic acid salt of formula (5).

The reaction solvent is preferably an aprotic polar organic solvent, examples of which include N,N-dimethylformamide, N,N-dimethylacetamide, N-methylpyrrolidone, 1,3-dimethyl-2-imidazolidinone, dimethylsulfoxide, tetrahydrofuran and dioxane. From the standpoint of the ease of removing the reaction solvent following the reaction, N,N-dimethylformamide, N,N-dimethylacetamide, tetrahydrofuran and dioxane are preferred.

The reaction temperature, taking into account the types of solvent and catalyst used and the types of starting compounds, should be a temperature between the melting point and boiling point of the solvent at which the reaction is capable of proceeding, and is generally from about 50°C to about 100°C. The reaction time differs according to the reaction conditions and therefore cannot be strictly specified, but is generally from 0.1 to 100 hours.

Following reaction completion, the arylsulfonic acid salt of formula (1') is recovered by filtration, distilling off the reaction solvent or the like, and the recovered salt is protonated with, for example, a cation exchange resin to give an arylsulfonic acid compound of formula (1).

Preferred examples of the arylsulfonic acid compound of the invention include, but are not limited to, the following: 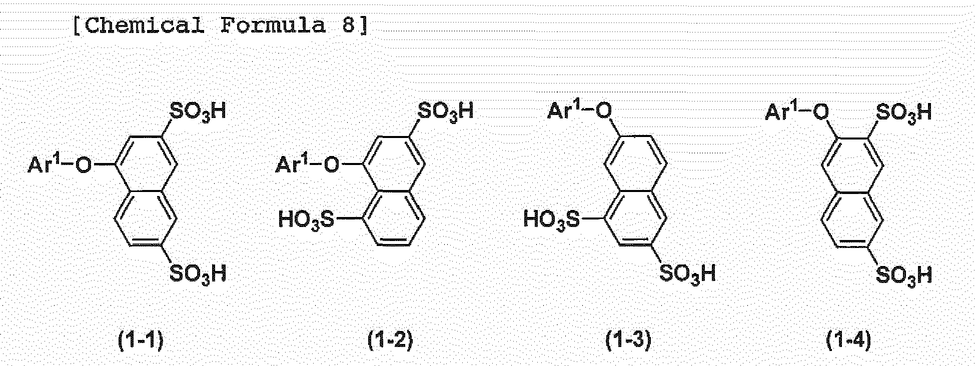 (wherein Ar¹ is as defined above).

### [Charge-Transporting Varnish]

The charge-transporting varnish of the invention includes the arylsulfonic acid compound of the invention as a dopant, and also includes a charge-transporting substance and an organic solvent.

As used herein, "charge transportability" is synonymous with electrical conductivity, and means the same as hole transportability. The charge-transporting substance may itself have charge transportability, or may have charge transportability when used together with a dopant. The charge-transporting varnish may itself have charge transportability, or a solid film obtained therefrom may have charge transportability.

This charge-transporting substance may be one that has hitherto been used in, for example, the organic EL field, and is exemplified by various types of hole-transporting substances, including aniline derivatives (arylamine derivatives) such as oligoaniline derivatives, N,N'-diarylbenzidine derivatives and N,N,N',N'-tetraarylbenzidine derivatives; thiophene derivatives such as oligothiophene derivatives, thienothiophene derivatives and thienobenzothiophene derivatives; and pyrrole derivatives such as oligopyrroles. Of these, aniline derivatives and thiophene derivatives are preferred, with aniline derivatives being more preferred.

In light of such considerations as suppressing precipitation of the charge-transporting substance and improving the application properties of the varnish, the molecular weight of the charge-transporting substance is preferably not more than 9,000, more preferably not more than 8,000, even more preferably not more than 7,000, still more preferably not more than 6,000, and yet more preferably not more than 5,000. On the other hand, to obtain a thin-film having a higher charge-transportability, the molecular weight is preferably at least 300. To prevent separation of the charge-transporting substance when it has been rendered into a thin-film, the charge-transporting substance preferably does not have a molecular weight distribution (dispersity = 1); i.e., it preferably has a single molecular weight.

A charge-transporting substance composed of the aniline derivative of formula (6) below is especially preferred in the invention.

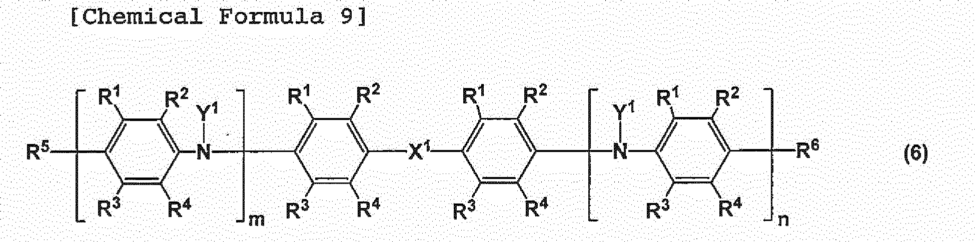

In formula (6), X¹ is -NY¹-, -O-, -S-, -(CR⁷R⁸)_{L}- or a single bond. When m or n is 0, X¹ is -NY¹-.

Each Y¹ is independently a hydrogen atom, an alkyl group of 1 to 20 carbon atoms, alkenyl group of 2 to 20 carbon atoms or alkynyl group of 2 to 20 carbon atoms which may be substituted with Z¹, or an aryl group of 6 to 20 carbon atoms or heteroaryl group of 2 to 20 carbon atoms which may be substituted with Z².

The alkyl group of 1 to 20 carbon atoms may be linear, branched or cyclic. Examples include linear or branched alkyl groups of 1 to 20 carbon atoms, such as methyl, ethyl, n-propyl, isopropyl, n-butyl, isobutyl, s-butyl, t-butyl, n-pentyl, n-hexyl, n-heptyl, n-octyl, n-nonyl and n-decyl; and cyclic alkyl groups of 3 to 20 carbon atoms such as cyclopropyl, cyclobutyl, cyclopentyl, cyclohexyl, cyclobutyl, cyclooctyl, cyclononyl, cyclodecyl, bicyclobutyl, bicyclopentyl, bicyclohexyl, bicycloheptyl, bicyclooctyl, bicyclononyl and bicyclodecyl.

Examples of alkenyl groups of 2 to 20 carbon atoms include ethenyl, n-1-propenyl, n-2-propenyl, 1-methylethenyl, n-1-butenyl, n-2-butenyl, n-3-butenyl, 2-methyl-1-propenyl, 2-methyl-2-propenyl, 1-ethylethenyl, 1-methyl-1-propenyl, 1-methyl-2-propenyl, n-1-pentenyl, n-1-decyl and n-1-eicosenyl.

Examples of alkynyl groups of 2 to 20 carbons include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl and n-1-eicosynyl.

Examples of aryl groups of 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl.

Examples of heteroaryl groups of 2 to 20 carbon atoms include 2-thienyl, 3-thienyl, 2-furanyl, 3-furanyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl.

R⁷ and R⁸ are each independently a hydrogen atom, a halogen atom, a nitro group, a cyano group, an amino group, an aldehyde group, a hydroxyl group, a thiol group, a sulfonic acid group, a carboxylic acid group, an alkyl group of 1 to 20 carbon atoms, alkenyl group of 2 to 20 carbon atoms or alkynyl group of 2 to 20 carbons which may be substituted with Z¹, an aryl group of 6 to 20 carbon atoms or heteroaryl group of 2 to 20 carbon atoms which may be substituted with Z², -NHY², -NY³Y⁴, -C(O)Y⁵, -OY⁶, -SY⁷, -SO₃Y⁸, -C(O)OY⁹, -OC(O)Y¹⁰, -C(O)NHY¹¹ or -C(O)NY¹²Y¹³. Y² to Y¹³ are each independently an alkyl group of 1 to 20 carbon atoms, alkenyl group of 2 to 20 carbon atoms or alkynyl group of 2 to 20 carbon atoms which may be substituted with Z¹, or an aryl group of 6 to 20 carbon atoms or heteroaryl group of 2 to 20 carbon atoms which may be substituted with Z².

Examples of the halogen atom include fluorine, chlorine, bromine and iodine atoms. The alkyl groups, alkenyl groups, alkynyl groups, aryl groups and heteroaryl groups of R⁷ to R⁸ and Y² to Y¹³ are exemplified by the same groups as mentioned above.

Of these, R⁷ and R⁸ are preferably a hydrogen atom, an alkyl group of 1 to 20 carbon atoms which may be substituted with Z¹, more preferably a hydrogen atom or a methyl group which may be substituted with Z¹, and most preferably a hydrogen atom.

L represents the number of repeating units of a divalent alkylene group of the formula -(CR⁷R⁸)-, this being an integer from 1 to 20, preferably from 1 to 10, more preferably from 1 to 5, still more preferably 1 or 2, and most preferably 1. When L is 2 or more, the plurality of R⁷ groups may be mutually the same or different, and the plurality of R⁸ may likewise be mutually the same or different.

In particular, X¹ is preferably -NY¹- or a single bond. Y¹ is preferably a hydrogen atom or an alkyl group of 1 to 20 carbon atoms which may be substituted with Z¹, more preferably a hydrogen atom or a methyl group which may be substituted with Z¹, and most preferably a hydrogen atom.

R¹ to R⁶ are each independently a hydrogen atom, a halogen atom, a nitro group, a cyano group, an amino group, an aldehyde group, a hydroxyl group, a thiol group, a sulfonic acid group, a carboxylic acid group, an alkyl group of 1 to 20 carbon atoms, alkenyl group of 2 to 20 carbon atoms or alkynyl group of 2 to 20 carbon atoms which may be substituted with Z¹, an aryl group of 6 to 20 carbon atoms or heteroaryl group of 2 to 20 carbon atoms which may be substituted with Z², -NHY², -NY³Y⁴, -C(O)Y⁵, -OY⁶, -SY⁷, - SO₃Y⁸, -C(O)OY⁹, -OC(O)Y¹⁰, -C(O)NHY¹¹ or -C(O)NY¹²Y¹³ (wherein Y² to Y¹³ are as defined above). These halogen atoms, alkyl groups, alkenyl groups, alkynyl groups, aryl groups and heteroaryl groups are exemplified by the same groups as mentioned above.

In particular, in formula (6), R¹ to R⁴ are each preferably a hydrogen atom, a halogen atom, an alkyl group of 2 to 20 carbon atoms which may be substituted with Z¹, or an aryl group of 6 to 14 carbon atoms which may be substituted with Z²; are each more preferably a hydrogen atom, a fluorine atom or an alkyl group of 1 to 10 carbon atoms which may be substituted with fluorine atoms; and are most preferably all hydrogen atoms.

R⁵ and R⁶ are each preferably a hydrogen atom, a halogen atom, an alkyl group of 1 to 10 carbon atoms which may be substituted with Z¹, an aryl group of 6 to 14 carbon atoms which may be substituted with Z², or a diphenylamino group which may be substituted with Z² (a -NY³Y⁴ group wherein Y³ and Y⁴ are phenyl groups which may be substituted with Z²); are each more preferably a hydrogen atom, a fluorine atom or a diphenylamino group which may be substituted with fluorine atoms; and are still more preferably both hydrogen atoms or both diphenylamino groups.

Of these, combinations in which R¹ to R⁴ are hydrogen atoms, fluorine atoms or alkyl groups of 1 to 10 carbon atoms which may be substituted with fluorine atoms, R⁵ and R⁶ are hydrogen atoms, fluorine atoms or diphenylamino groups which may be substituted with fluorine atoms, X¹ is -NY¹- or a single bond, and Y¹ is a hydrogen atom or a methyl group are preferred. Combinations in which R¹ to R⁴ are hydrogen atoms, R⁵ and R⁶ are both hydrogen atoms or diphenylamino groups, and X¹ is -NH- or a single bond are more preferred.

In formula (6), the letters m and n independently represent an integer of 0 or more and satisfy the condition 1 ≤ m+n ≤ 20. Taking into account the balance between the charge transportability of the resulting thin-film and the solubility of the aniline derivative, m and n preferably satisfy the condition 2 ≤ m+n ≤ 8, more preferably satisfy the condition 2 ≤ m+n ≤ 6, and still more preferably satisfy the condition 2 ≤ m+n ≤ 4.

The alkyl groups, alkenyl groups and alkynyl groups of Y¹ to Y¹³ and R¹ to R⁸ may be substituted with Z¹, which is a halogen atom, a nitro group, a cyano group, an amino group, an aldehyde group, a hydroxyl group, a thiol group, a sulfonic acid group, a carboxylic acid group, or an aryl group of 6 to 20 carbon atoms or heteroaryl group of 2 to 20 carbon atoms which may be substituted with Z³. The aryl groups and heteroaryl groups of Y¹ to Y¹³ and R¹ to R⁸ may be substituted with Z², which is a halogen atom, a nitro group, a cyano group, an amino group, an aldehyde group, a hydroxyl group, a thiol group, a sulfonic acid group, a carboxylic acid group, or an alkyl group of 1 to 20 carbon atoms, alkenyl group of 2 to 20 carbon atoms or alkynyl group of 2 to 20 carbon atoms which may be substituted with Z³. These groups may be additionally substituted with Z³, which is a halogen atom, a nitro group, a cyano group, an amino group, an aldehyde group, a hydroxyl group, a thiol group, a sulfonic acid group, or a carboxylic acid group (the halogen atom being exemplified in the same way as above).

In particular, in Y¹ to Y¹³ and R¹ to R⁸, the substituent Z¹ is preferably a halogen atom or an aryl group of 6 to 20 carbon atoms which may be substituted with Z³, more preferably a halogen atom or a phenyl group which may be substituted with Z³, and most preferably does not exist (i.e., is non-substituting).

The substituent Z² is preferably a halogen atom or an alkyl group of 1 to 20 carbon atoms which may be substituted with Z³, more preferably a halogen atom or an alkyl group of 1 to 4 carbon atoms which may be substituted with Z³, and most preferably does not exist (i.e., is non-substituting).

Also, Z³ is preferably a halogen atom, more preferably fluorine, and most preferably does not exist (i.e., is non-substituting).

In Y¹ to Y¹³ and R¹ to R⁸, the number of carbon atoms on the alkyl group, alkenyl group and alkynyl group is preferably 10 or less, more preferably 6 or less, and even more preferably 4 or less.

The number of carbons on the aryl group and heteroaryl group is preferably 14 or less, more preferably 10 or less, and even more preferably 6 or less.

The aniline derivative of formula (6) has a molecular weight which, from the standpoint of increasing its solubility, is preferably not more than 4,000, and more preferably not more than 3,000.

Examples of methods for synthesizing the aniline derivative used in the invention include, but are not particularly limited to, the methods described in Bulletin of Chemical Society of Japan, 67, pp. 1749-1752 (1994); Synthetic Metals, 84, pp. 119-120 (1997); Thin Solid Films, 520 (24), pp. 7157-7163 (2012), WO 2008/032617, WO 2008-032616, WO 2008-129947 and WO 2014/148415.

Examples of preferred aniline derivatives in this invention include, but are not limited to, those shown below. In the following formulas, "DPA" stands for a diphenylamino group, "Ph" stands for a phenyl group, and "TPA" stands for a p-(diphenylamino)phenyl group.

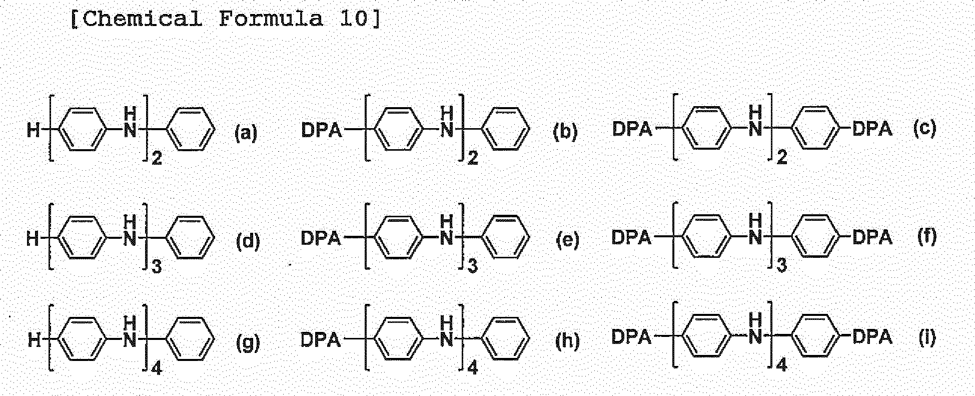

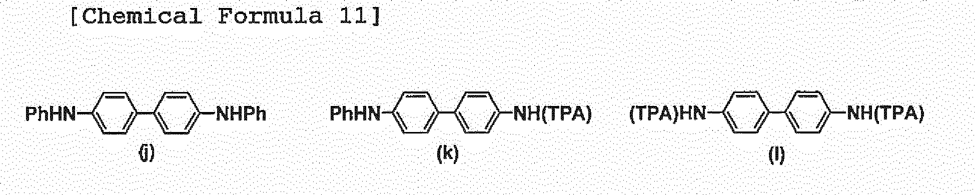

The charge-transporting varnish of the invention may include other dopants in addition to the arylsulfonic acid of the invention. From the standpoint of compatibility with the arylsulfonic acid compound of the invention and the charge transportability of the resulting thin-film, preferred examples include heteropolyacid compounds.

"Heteropolyacid compound" refers to a polyacid having a structure in which a heteroatom is positioned at the center of the molecule--typically the Keggin-type chemical structure shown in formula (A) or the Dawson-type chemical structure shown in formula (B), and which is obtained by the condensation of an isopolyacid that is an oxoacid of vanadium (V), molybdenum (Mo), tungsten (W) or the like with an oxoacid of a different element. Examples of such oxoacids of a different element include primarily oxoacids of silicon (Si), phosphorus (P) and arsenic (As).

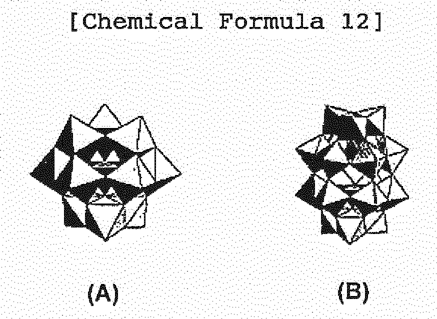

Examples of heteropolyacid compounds include phosphomolybdic acid, silicomolybdic acid, phosphotungstic acid, silicotungstic acid and phosphotungstomolybdic acid. These may be used singly, or two or more may be used in combination. The heteropolyacid compound used in this invention may be acquired as a commercial product or may be synthesized by a known method.

In particular, to enhance the brightness characteristics in cases where the resulting thin-film is to be used as a hole injection layer in an organic EL device, phosphotungstic acid and phosphomolybdic acid are preferred, with phosphotungstic acid being best.

When using the arylsulfonic acid compound of the invention to prepare a charge-transporting varnish, the amount of arylsulfonic acid compound, expressed as a molar ratio relative to unity (1) for the charge-transporting substance, is about 0.5 to 10, and preferably about 0.75 to 5.

The charge-transporting varnish of the invention may include an organosilane compound for such purposes as to adjust the application properties of the varnish onto a substrate, and to control the ionization potential of the resulting thin-film.

The organosilane compound is exemplified by dialkoxysilane compounds, trialkoxysilane compounds and tetraalkoxysilane compounds. These may be used singly or two or more may be used in combination.

In particular, a dialkoxysilane compound or a trialkoxysilane compound is preferred, and a trialkoxysilane compound is more preferred, as the organosilane compound.

The tetraalkoxysilane compound, trialkoxysilane compound and dialkoxysilane compound are exemplified by compounds of formulas (7) to (9) below.

Si(OR⁹)₄ (7)

SiR¹⁰(OR⁹)₃ (8)

Si(R¹⁰)₂(OR⁹)₂ (9)

In these formulas, each R⁹ is independently an alkyl group of 1 to 20 carbon atoms, alkenyl group or 2 to 20 carbon atoms or alkynyl group of 2 to 20 carbon atoms which may be substituted with Z⁴, or an aryl group of 6 to 20 carbon atoms or heteroaryl group of 2 to 20 carbon atoms which may be substituted with Z⁵; and each R¹⁰ is independently an alkyl group of 1 to 20 carbon atoms, alkenyl group or 2 to 20 carbon atoms or alkynyl group of 2 to 20 carbon atoms which may be substituted with Z⁶, or an aryl group of 6 to 20 carbon atoms or heteroaryl group of 2 to 20 carbon atoms which may be substituted with Z⁷.

Z⁴ is a halogen atom or an aryl group of 6 to 20 carbon atoms or heteroaryl group of 2 to 20 carbon atoms which may be substituted with Z⁸; Z⁵ is a halogen atom or an alkyl group of 1 to 20 carbon atoms, alkenyl group of 2 to 20 carbon atoms or alkynyl group of 2 to 20 carbon atoms which may be substituted with Z⁸.

Z⁶ is a halogen atom, an aryl group of 6 to 20 carbon atoms or heteroaryl group of 2 to 20 carbon atoms which may be substituted with Z⁸, an epoxycyclohexyl group, a glycidoxy group, a methacryloxy group, an acryloxy group, a ureido group (-NHCONH₂), a thiol group, an isocyanato group (-NCO), an amino group, a -NHY¹⁴ group or a -NY¹⁵Y¹⁶ group.

Z⁷ is a halogen atom, an alkyl group of 1 to 20 carbon atoms, alkenyl group of 2 to 20 carbon atoms or alkynyl group of 2 to 20 carbon atoms which may be substituted with Z⁸, an epoxycyclohexyl group, a glycidoxy group, a methacryloxy group, an acryloxy group, a ureido group (-NHCONH₂), a thiol group, an isocyanato group (-NCO), an amino group, a -NHY¹⁴ group or a -NY¹⁵Y¹⁶ group, with Y¹⁴ to Y¹⁶ being each independently an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which may be substituted with Z⁸.

Z⁸ is a halogen atom, an amino group, a nitro group, a cyano group or a thiol group.

The halogen atoms, alkyl groups of 1 to 20 carbon atoms, alkenyl groups of 2 to 20 carbon atoms, alkynyl groups of 2 to 20 carbon atoms, aryl groups of 6 to 20 carbon atoms and heteroaryl groups of 2 to 20 carbon atoms in formulas (7) to (9) are exemplified in the same way as above.

The number of carbon atoms on the alkyl groups, alkenyl groups and alkynyl groups in R⁹ and R¹⁰ is preferably 10 or less, more preferably 6 or less, and even more preferably 4 or less.

The number of carbon atoms on the aryl groups and heteroaryl groups is preferably 14 or less, more preferably 10 or less, and even more preferably 6 or less.

R⁹ is preferably an alkyl group of 1 to 20 carbon atoms or alkenyl group of 2 to 20 carbon atoms which may be substituted with Z⁴ or an aryl group of 6 to 20 carbon atoms which may be substituted with Z⁵; more preferably an alkyl group of 1 to 6 carbon atoms or alkenyl group of 2 to 6 carbon atoms which may be substituted with Z⁴ or a phenyl group which may be substituted with Z⁵; even more preferably an alkyl group of 1 to 4 carbon atoms which may be substituted with Z⁴ or a phenyl group which may be substituted with Z⁵_{;} and still more preferably a methyl or ethyl group which may be substituted with Z⁴.

R¹⁰ is preferably an alkyl group of 1 to 20 carbon atoms which may be substituted with Z⁶ or an aryl group of 6 to 20 carbon atoms which may be substituted with Z⁷; more preferably an alkyl group of 1 to 10 carbon atoms which may be substituted with Z⁶ or an aryl group of 6 to 14 carbon atoms which may be substituted with Z⁷; still more preferably an alkyl group of 1 to 6 carbon atoms which may be substituted with Z⁶ or an aryl group of 6 to 10 carbon atoms which may be substituted with Z⁷; and still more preferably an alkyl group of 1 to 4 carbon atoms which may be substituted with Z⁶ or a phenyl group which may be substituted with Z⁷.

When there are a plurality of R⁹ groups, they may all be the same or different. When there are a plurality of R¹⁰ groups, they may all be the same or different.

Z⁴ is preferably a halogen atom or an aryl group of 6 to 20 carbon atoms which may be substituted with Z⁸, more preferably a fluorine atom or a phenyl group which may be substituted with Z⁸, and most preferably does not exist (i.e., is non-substituting).

Z⁵ is preferably a halogen atom or an alkyl group of 6 to 20 carbon atoms which may be substituted with Z⁸, more preferably a fluorine atom or an alkyl group of 1 to 10 carbon atoms which may be substituted with Z⁸, and most preferably does not exist (i.e., is non-substituting).

Z⁶ is preferably a halogen atom, a phenyl group which may be substituted with Z⁸, a furanyl group which may be substituted with Z⁸, an epoxycyclohexyl group, a glycidoxy group, a methacryloxy group, an acryloxy group, a ureido group, a thiol group, an isocyanate group, an amino group, a phenylamino group which may be substituted with Z⁸, or a diphenylamino group which may be substituted with Z⁸; more preferably a halogen atom; and even more preferably a fluorine atom or does not exist (i.e., is non-substituting).

Z⁷ is preferably a halogen atom, an alkyl group of 1 to 20 carbon atoms which may be substituted with Z⁸, a furanyl group which may be substituted with Z⁸, an epoxycyclohexyl group, a glycidoxy group, a methacryloxy group, an acryloxy group, a ureido group, a thiol group, an isocyanato group, an amino group, a phenylamino group which may be substituted with Z⁸, or a diphenylamino group which may be substituted with Z⁸; more preferably a halogen atom; and even more preferably a fluorine atom or does not exist (i.e., is non-substituting).

Z⁸ is preferably a halogen atom, and more preferably a fluorine atom or does not exist (i.e., is non-substituting).

Examples of organosilane compounds that may be used in the invention include, but are not limited to, the following.

Specific examples of dialkoxysilane compounds include dimethyldimethoxysilane, dimethyldiethoxysilane, methylethyldimethoxysilane, diethyldimethoxysilane, diethyldiethoxysilane, methylpropyldimethoxysilane, methylpropyldiethoxysilane, diisopropyldimethoxysilane, phenylmethyldimethoxysilane, vinylmethyldimethoxysilane, 3-glycidoxypropylmethyldimethoxysilane, 3-glycidoxypropylmethyldiethoxysilane, 3-(3,4-epoxycyclohexyl)ethylmethyldimethoxysilane, 3-methacryloxypropylmethyldimethoxysilane, 3-methacryloxypropylmethyldiethoxysilane, 3-mercaptopropylmethyldimethoxysilane, 3-aminopropylmethyldiethoxysilane, N-(2-aminoethyl)aminopropylmethyldimethoxysilane and 3,3,3-trifluoropropylmethyldimethoxysilane.

Specific examples of trialkoxysilane compounds include methyltrimethoxysilane, methyltriethoxysilane, ethyltrimethoxysilane, ethyltriethoxysilane, propyltrimethoxysilane, propyltriethoxysilane, butyltrimethoxysilane, butyltriethoxysilane, pentyltrimethoxysilane, pentyltriethoxysilane, heptyltrimethoxysilane, heptyltriethoxysilane, octyltrimethoxysilane, octyltriethoxysilane, dodecyltrimethoxysilane, dodecyltriethoxysilane, hexadecyltrimethoxysilane, hexadecyltriethoxysilane, octadecyltrimethoxysilane, octadecyltriethoxysilane, phenyltrimethoxysilane, phenyltriethoxysilane, vinyltrimethoxysilane, vinyltriethoxysilane, 3-aminopropyltrimethoxysilane, 3-aminopropyltriethoxysilane, 3-glycidoxypropyltrimethoxysilane, 3-glycidoxypropyltriethoxysilane, 3-methacryloxypropyltrimethoxysilane, 3-methacryloxypropyltriethoxysilane, triethoxy(4-(trifluoromethyl)phenyl)silane, dodecyltriethoxysilane, 3,3,3-trifluoropropyltrimethoxysilane, (triethoxysilyl)cyclohexane, perfluorooctylethyltriethoxysilane, triethoxyfluorosilane, tridecafluoro-1,1,2,2,-tetrahydrooctyltriethoxysilane, pentafluorophenyltrimethoxysilane, pentafluorophenyltriethoxysilane, 3-(heptafluoroisopropoxy)propyltriethoxysilane, heptadecafluoro-1,1,2,2-tetrahydrodecyltriethoxysilane, triethoxy-2-thienylsilane and 3-(triethoxysilyl)furan.

Specific examples of tetraalkoxysilane compounds include tetramethoxysilane, tetraethoxysilane and tetrapropoxysilane.

Of these, 3,3,3-trifluoropropylmethyldimethoxysilane, triethoxy(4-(trifluoromethyl)phenyl)silane, 3,3,3-trifluoropropyltrimethoxysilane, perfluorooctylethyltriethoxysilane, pentafluorophenyltrimethoxysilane and pentafluorophenyltriethoxysilane are preferred.

To maintain a high charge transportability in the resulting thin-film, the content of the organosilane compound in the charge-transporting varnish of the invention, based on the total mass of the charge-transporting substance and the dopant, is preferably about 0.5 to 40 mass%, more preferably about 0.8 to 30 mass%, and still more preferably 1 to 20 mass%.

Highly solvating solvents which are capable of dissolving well the charge-transporting substance and the dopant may be used as the organic solvent employed when preparing the charge-transporting varnish.

Examples of such highly solvating solvents that may be used include organic solvents such as N,N-dimethylformamide, N,N-dimethylacetamide, N-methylpyrrolidone, 1,3-dimethyl-2-imidazolidinone and diethylene glycol monomethyl ether. These solvents may be used singly, or two or more may be used in admixture. The amount thereof may be set to 5 to 100 mass%, based on the overall solvent used in the varnish.

To obtain a thin-film having a high flatness, both the charge-transporting substance and the dopant are preferably in a state where they are either completely dissolved or uniformly dispersed in the solvent; and more preferably in a state where they are completely dissolved.

In the practice of the invention, by including in the varnish at least one high-viscosity organic solvent having a viscosity at 25°C of 10 to 200 mPa·s, especially 35 to 150 mPa·s, and a boiling point at standard pressure (atmospheric pressure) of 50 to 300°C, especially 150 to 250°C, the viscosity of the varnish is easily adjusted, thus making it easier to prepare a varnish which reproducibly gives a thin-film of high flatness and is suitable for the coating method.

Examples of high-viscosity organic solvents include, but are not particularly limited to, cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol and hexylene glycol. These solvents may be used singly, or two or more may be used in admixture.

The amount of high-viscosity organic solvent added as a proportion of the overall solvent used in the varnish of the invention is preferably within a range where no precipitation of solids occurs. The amount of such addition is preferably 5 to 80 mass%, provided that no precipitation of solids occurs.

In addition, other solvents may be admixed in a proportion with respect to the overall solvent used in the varnish of 1 to 90 mass%, and preferably 1 to 50 mass%, for such purposes as to enhance the substrate wettability by the varnish, adjust the solvent surface tension, adjust the polarity, and adjust the boiling point.

Examples of such solvents include, but are not limited to, ethylene glycol monobutyl ether, diethylene glycol diethyl ether, diethylene glycol dimethyl ether, diethylene glycol monoethyl ether acetate, diethylene glycol monobutyl ether acetate, dipropylene glycol monomethyl ether, propylene glycol monomethyl ether acetate, diethylene glycol monoethyl ether, diacetone alcohol, γ-butyrolactone, ethyl lactate, n-hexyl acetate and propylene glycol monomethyl ether. These solvents may be used singly, or two or more may be used in admixture.

The viscosity of the inventive varnish is set as appropriate for the thickness and other properties of the thin-film to be produced and the solids concentration of the varnish, but is generally from 1 to 50 mPa·s at 25°C.

The solids concentration of the charge-transporting varnish of this invention is set as appropriate based on such considerations as the viscosity, surface tension and other properties of the varnish and the thickness and other properties of the thin-film to be produced, and is generally about 0.1 to 10.0 mass%. To improve the application properties of the varnish, the solids concentration of the varnish is preferably about 0.5 to 5.0 mass%, and more preferably 1.0 to 3.0 mass%.

The method of preparing the charge-transporting varnish is exemplified by, but not particularly limited to, a method in which the arylsulfonic acid compound of the invention is first dissolved in a solvent, after which a charge-transporting substance is added to the solution; and a method in which a mixture of the arylsulfonic acid compound of the invention and a charge-transporting substance is dissolved in a solvent. In cases where there are a plurality of organic solvents, either the arylsulfonic acid compound of the invention and the charge-transporting substance may first be dissolved in a solvent that dissolves them well, after which the other solvent or solvents may be added thereto, or the arylsulfonic acid compound of the invention and the charge-transporting substance may be dissolved one after the other or at the same time in a mixed solvent of the plurality of organic solvents.

In this invention, from the standpoint of reproducibly obtaining high-flatness thin-films, after the dopant consisting of the arylsulfonic acid compound of the invention, the charge-transporting substance and the like have been dissolved in an organic solvent, it is desirable for the charge-transporting varnish to be filtered using, for example, a submicron order filter.

A charge-transporting thin-film can be formed on a substrate by applying the charge-transporting varnish of the invention on a substrate and baking the applied varnish.

In general, to reproducibly obtain high-flatness thin-films, it is desirable to carry out firing in two steps: pre-firing for the purpose of primarily drying the liquid film (applied film) and imparting flatness, and main firing for such purposes as to remove the solvent and impart higher charge transportability. At this time, it is more desirable to maintain a uniform liquid film state during pre-firing.

The charge-transporting varnish of the invention, by including the arylsulfonic acid compound of the invention, suppresses agglomeration of the liquid film (applied film) coated onto a substrate, especially indium-tin oxide (ITO), making it possible to maintain a uniform liquid film state on the substrate. Hence, by using the charge-transporting varnish of the invention to form a thin-film by a two-step bake, thin-films of excellent flatness and charge transportability can be reproducibly obtained.

The pre-bake temperature varies according to, for example, the types of solvent and charge-transporting substance used together, and thus cannot be strictly specified, but is generally between 50°C and 100°C. The main bake temperature differs depending on such factors as the types of solvent and charge-transporting substance used together and the degree of charge transportability to be imparted to the resulting thin-film and thus cannot be strictly specified, but is generally between 100°C and 260°C. In particular, when the resulting thin-film is to be used as a hole injection layer in an organic EL device, a temperature of about 180°C to 250°C is often used as the main bake temperature.

The thin-film, depending on the intended use thereof, may be formed in a one-step bake process consisting solely of a main bake.

Heating at the time of the bake process may be carried out using a suitable apparatus such as hot plate or an oven.

Examples of methods for applying the varnish include, but are not limited to, dipping, spin coating, transfer printing, roll coating, brush coating, ink-jet printing and spraying. It is preferable to adjust the viscosity and surface tension of the varnish according to the method of application.

When using the varnish of the invention, the bake atmosphere is not particularly limited. A thin-film having a uniform film-forming surface and a high charge transportability can be obtained not only in an open-air atmosphere, but even in an inert gas such as nitrogen or in a vacuum.

The thickness of the charge-transporting thin-film is not particularly limited. However, when the thin-film is to be used as a hole injection layer in an organic EL device, a film thickness of 5 to 200 nm is preferred. Methods for varying the film thickness include, for example, changing the solids concentration in the varnish and changing the amount of solution on the substrate during application. Here, "solids" typically refers to the charge-transporting substance and the dopant.

The charge-transporting thin-film of the invention can be suitably used as a hole injection layer in an organic EL device, although use as a charge-transporting functional layer such as a hole injecting and transporting layer is also possible.

### [Organic EL Device]

The organic EL device of the invention has a pair of electrodes and, between these electrodes, the above-described charge-transporting thin-film of the invention.

Typical organic EL device configurations include, but are not limited to, those of (a) to (f) below. In these configurations, where necessary, an electron-blocking layer or the like may be provided between the emissive layer and the anode, and a hole-blocking layer or the like may be provided between the emissive layer and the cathode. Alternatively, the hole injection layer, hole-transporting layer or hole injecting and transporting layer may also have the function of, for example, an electron-blocking layer; and the electron injection layer, electron-transporting layer or electron injecting and transporting layer may also have the function of, for example, a hole-blocking layer.
(a) anode/hole injection layer/hole-transporting layer/emissive layer/electron-transporting layer/electron injection layer/cathode
(b) anode/hole injection layer/hole-transporting layer/emissive layer/electron injecting and transporting layer/cathode
(c) anode/hole injecting and transporting layer/emissive layer/electron-transporting layer/electron injection layer/cathode
(d) anode/hole injecting and transporting layer/emissive layer/electron injecting and transporting layer/cathode
(e) anode/hole injection layer/hole-transporting layer/emissive layer/cathode
(f) anode/hole injecting and transporting layer/emissive layer/cathode

As used herein, "hole injection layer," "hole-transporting layer" and "hole injecting and transporting layer" refer to layers which are formed between the emissive layer and the anode, and which have the function of transporting holes from the anode to the emissive layer. When only one layer of hole-transporting material is provided between the emissive layer and the anode, this is a "hole injecting and transporting layer"; when two or more layers of hole-transporting material are provided between the emissive layer and the anode, the layer that is closer to the anode is a "hole injection layer" and the other layer is a "hole-transporting layer." In particular, thin-films having not only an ability to receive holes from the anode but also an excellent ability to inject holes into, respectively, the hole-transporting layer and the emissive layer may be used as the hole injection layer and the hole injecting and transporting layer.

In addition, "electron injection layer," "electron-transporting layer" and "electron injecting and transporting layer" refer to layers which are formed between the emissive layer and the cathode, and which have the function of transporting electrons from the cathode to the emissive layer. When only one layer of electron-transporting material is provided between the emissive layer and the cathode, this is an "electron injecting and transporting layer"; when two or more layers of electron-transporting material are provided between the emissive layer and the cathode, the layer that is closer to the cathode is an "electron injection layer" and the other layer is an "electron-transporting layer."

The "emissive layer" is an organic layer having a light-emitting function. When a doping system is used, this layer includes a host material and a dopant material. The function of the host material is primarily to promote the recombination of electrons and holes, and to confine the resulting excitons within the emissive layer. The function of the dopant material is to cause the excitons obtained by recombination to efficiently emit light. In the case of a phosphorescent device, the host material functions primarily to confine within the emissive layer the excitons generated by the dopant.

The materials and methods employed to fabricate an organic EL device using the charge-transporting varnish of the invention are exemplified by, but not limited to, those described below.

The electrode substrate to be used is preferably cleaned beforehand by liquid washing with, for example, a cleaning agent, alcohol or pure water. When the substrate is an anode substrate, it is preferably subjected to surface treatment such as UV/ozone treatment or oxygen-plasma treatment just prior to use. However, surface treatment need not be carried out if the anode material is composed primarily of organic substances.

A method of fabricating an inventive organic EL device in which a thin-film obtained from the charge-transporting varnish of the invention serves as a hole injection layer is described below by way of illustration.

In the manner described above, a hole injection layer is formed on an electrode by applying the charge-transporting varnish of the invention onto an anode substrate and baking. A hole-transporting layer, emissive layer, electron-transporting layer, electron injection layer and cathode are then provided in this order on the hole injection layer. The hole-transporting layer, emissive layer, electron-transporting layer and electron injection layer may be formed by a vapor deposition process or a coating process (wet process), depending on the properties of the material to be used.

Illustrative examples of anode materials include transparent electrodes such as indium-tin oxide (ITO) and indium-zinc oxide (IZO), and metal anodes made of a metal such as aluminum or an alloy of such a metal. An anode material on which planarizing treatment has been carried out is preferred. Use can also be made of polythiophene derivatives and polyaniline derivatives having high charge transportability.

Examples of other metals making up the metal anode include, but are not limited to, scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and alloys thereof.

Specific examples of hole-transporting layer-forming materials include the following hole-transporting low-molecular-weight materials: triarylamines such as (triphenylamine) dimer derivatives, [(triphenylamine) dimer] spirodimer, N,N'-bis(naphthalen-1-yl)-N,N'-bis(phenyl)benzidine (α-NPD), N,N'-bis(naphthalen-2-yl)-N,N'-bis(phenyl)benzidine, N,N'-bis(3-methylphenyl)-N,N'-bis(phenyl)benzidine, N,N'-bis(3-methylphenyl)-N,N'-bis(phenyl)-9,9-spirobifluorene, N,N'-bis(naphthalen-1-yl)-N,N'-bis(phenyl)-9,9-spirobifluorene, N,N'-bis(3-methylphenyl)-N,N'-bis(phenyl)-9,9-dimethylfluorene, N,N'-bis(naphthalen-1-yl)-N,N'-bis(phenyl)-9,9-dimethylfluorene, N,N'-bis(3-methylphenyl)-N,N'-bis(phenyl)-9,9-diphenylfluorene, N,N'-bis(naphthalen-1-yl)-N,N'-bis(phenyl)-9,9-diphenylfluorene, N,N'-bis(naphthalen-1-yl)-N,N'-bis(phenyl)-2,2'-dimethylbenzidine, 2,2',7,7'-tetrakis(N,N-diphenylamino)-9,9-spirobifluorene, 9,9-bis[4-(N,N-bisbiphenyl-4-ylamino)phenyl]-9H-fluorene, 9,9-bis[4-(N,N-bisnaphthalen-2-ylamino)phenyl]-9H-fluorene, 9.9-bis[4-(N-naphthalen-1-yl-N-phenylamino)phenyl]-9H-fluorene, 2,2',7,7'-tetrakis[N-naphthalenyl(phenyl)amino]-9,9-spirobifluorene, N,N'-bis(phenanthren-9-yl)-N,N'-bis(phenyl)benzidine, 2,2'-bis[N,N-bis(biphenyl-4-yl)amino]-9,9-spirobifluorene, 2,2'-bis(N,N-diphenylamino)-9,9-spirobifluorene, di[4-(N,N-di(p-tolyl)amino)phenyl]cyclohexane, 2,2',7,7'-tetra(N,N-di(p-tolyl))amino-9,9-spirobifluorene, N,N,N',N'-tetranaphthalen-2-ylbenzidine, N,N,N',N'-tetra(3-methylphenyl)-3,3'-dimethylbenzidine, N,N'-di(naphthalenyl)-N,N'-di(naphthalen-2-yl)benzidine, N,N,N',N'-tetra(naphthalenyl)benzidine, N,N'-di(naphthalen-2-yl)-N,N'-diphenylbenzidine-1-4-diamine, N¹,N⁴- diphenyl-N¹,N⁴-di(m-tolyl)benzene-1,4-diamine, N², N², N⁶, N⁶-tetraphenylnaphthalene-2,6-diamine, tris(4-(quinolin-8-yl)phenyl)amine, 2,2'-bis(3-(N,N-di(p-tolyl)amino)phenyl)biphenyl, 4,4',4"-tris[3-methylphenyl(phenyl)amino]triphenylamine (m-MTDATA) and 4,4',4"-tris[1-naphthyl(phenyl)amino]triphenylamine (1-TNATA); and oligothiophenes such as 5,5"-bis-{4-[bis(4-methylphenyl)amino]phenyl}-2,2':5',2"-terthiophene (BMA-3T).

Specific examples of emissive layer-forming materials include tris(8-quinolinolate) aluminum(III) (Alq₃), bis(8-quinolinolate) zinc(II) (Znq₂), bis(2-methyl-8-quinolinolate)-4-(p-phenylphenolate) aluminum(III) (BAlq),
4,4'-bis(2,2-diphenylvinyl)biphenyl, 9,10-di(naphthalon-2-yl)anthracene, 2-t-butyl-9,10-di(naphthalen-2-yl)anthracene, 2,7-bis[9,9-di(4-methylphenyl)fluoren-2-yl]-9,9-di(4-methylphenyl)fluorene, 2-methyl-9,10-bis(naphthalen-2-yl)anthracene, 2-(9,9-spirobifluoren-2-yl)-9,9-spirobifluorene, 2,7-bis(9,9-spirobifluoren-2-yl)-9,9-spirobifluorene, 2-[9,9-di(4-methylphenyl)fluoren-2-yl]-9,9-di(4-methylphenyl)fluorene,
2,2'-dipyrenyl-9,9-spirobifluorene, 1,3,5-tris(pyren-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)phenyl]amine, 10,10'-di(biphenyl-4-yl)-9,9'-bianthracene, N,N'-di(naphthalen-1-yl)-N,N'-diphenyl[1,1':4',1":4",1"'-quaterphenyl]-4,4"'-diamine, 4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluoren-2-yl)pyrene, 1-(7-(9,9'-bianthracen-10-yl)-9,9-dihexyl-9H-fluoren-2-yl)pyrene, 1,3-bis(carbazol-9-yl)benzene, 1,3,5-tris(carbazol-9-yl)benzene, 4,4',4"-tris(carbazol-9-yl)triphenylamine, 4,4'-bis(carbazol-9-yl)biphenyl (CBP), 4,4'-bis(carbazol-9-yl)-2,2'-dimethylbiphenyl, 2,7-bis(carbazol-9-yl)-9,9-dimethylfluorene, 2,2',7,7'-tetrakis(carbazol-9-yl)-9,9-spirobifluorene, 2,7-bis(carbazol-9-yl)-9,9-di(p-tolyl)fluorene, 9,9-bis[4-(carbazol-9-yl)phenyl]fluorene, 2,7-bis(carbazol-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazol-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazole, 9-(4-t-butylphenyl)-3,6-ditrityl-9H-carbazole, 9-(4-t-butylphenyl)-3,6-bis(9-(4-methoxyphenyl)-9H-fluoren-9-yl)-9H-carbazole, 2,6-bis(3-(9H-carbazol-9-yl)phenyl)pyridine, triphenyl(4-(9-phenyl-9H-fluoren-9-yl)phenyl)silane, 9,9-dimethyl-N,N-diphenyl-7-(4-(1-phenyl-1H-benzo[d]imidazol-2-yl)phenyl-9H-fluoren-2-amine, 3,5-bis(3-(9H-carbazol-9-yl)phenyl)pyridine, 9,9-spirobifluoren-2-yldiphenylphosphine oxide, 9,9'-(5-triphenylsilyl)-1,3-phenylene)bis(9H-carbazole), 3-(2,7-bis(diphenylphosphoryl)-9-phenyl-9H-fluoren-9-yl)-9-phenyl-9H-carbazole, 4,4,8,8,12,12-hexa(p-tolyl)-4H-8H-12H-12C-azadibenzo[cd,mn]-pyrene,
4,7-di(9H-carbazol-9-yl)-1,10-phenanthroline, 2,2'-bis(4-(carbazol-9-yl)phenyl)biphenyl, 2,8-bis(diphenylphosphoryl)dibenzo[b,d]thiophene, bis(2-methylphenyl)diphenylsilane, bis[3,5-di(9H-carbazol-9-yl)phenyl]diphenylsilane, 3,6-bis(carbazol-9-yl)-9-(2-ethylhexyl)-9H-carbazole, 3-(diphenylphosphoryl)-9-(4-(diphenylphosphoryl)phenyl)-9H-carbazole and 3,6-bis[(3,5-diphenyl)phenyl]-9-phenylcarbazole. It is also possible to form the emissive layer by co-vapor deposition of any of these materials with a light-emitting dopant.

Specific examples of light-emitting dopants include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino[9,9a,1gh]coumarin, quinacridone, N,N'-dimethylquinacridone, tris(2-phenylpyridine) iridium(III) (Ir(ppy)₃), bis(2-phenylpyridine)(acetylacetonate) iridium(III) (Ir(ppy)₂(acac)), tris[2-(p-tolyl]pyridine) iridium(III) (Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolate] zinc(II), N¹⁰,N¹⁰,N¹⁰,N¹⁰-tetra(p-tolyl)-9,9'-bianthracene-10,10'-diamine, N¹⁰,N¹⁰,N¹⁰,N¹⁰-tetraphenyl-9,9'-bianthracene-10,10'-diamine, N¹⁰,N¹⁰-diphenyl-N¹⁰,N¹⁰-dinaphthalenyl-9,9'-bianthracene-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)] iridium(III),
4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)tris(9,9-dimethyl-fluorenylene), 2,7-bis{2-Ephenyl(m-tolyl)amino]-9,9-dimethylfluoren-7-yl)-9,9-dimethylfluorene, N-(4-((E)-2-(6((E)-4-(diphenylamino)styryl)naphthalen-2-yl)vinyl)phenyl)-N-phenylbenzenamine, fac-iridium(III) tris(1-phenyl-3-methylbenzimidazolin-2-ylidene-C, C²), mer-iridium(III) tris(1-phenyl-3-methylbenzimidazolin-2-ylidene-C,C²) , 2,7-bis[4-(diphenylamino)styryl]-9,9-spirobifluorene, 6-methyl-2-(4-(9-(4-(6-methylbenzo[d]thiazol-2-yl)phenyl)-anthracen-10-yl)phenyl)benzo[d]thiazole, 1,4-di[4-(N,N-diphenyl)amino]styrylbenzene, 1,4-bis(4-(9H-carbazol-9-yl)styryl)benzene,
(E)-6-(4-(diphenylamino)styryl)-N,N-diphenylnaphthalen-2-amine, bis(2,4-difluorophenylpyridinato) (5-(pyridin-2-yl)-1H-tetrazolate) iridium(III),
bis(3-trifluoromethyl-5-(2-pyridyl)pyrazole) ((2,4-difluoro-benzyl)diphenylphosphinate) iridium(III),
bis(3-trifluoromethyl-5-(2-pyridyl)pyrazolate) (benzyl-diphenylphosphinate) iridium(III),
bis(1-(2,4-difluorobenzyl)-3-methylbenzimidazolium) (3-(trifluoromethyl)-5-(2-pyridyl)-1,2,4-triazolate) iridium(III), bis(3-trifluoromethyl-5-(2-pyridyl)pyrazolate)(4',6'-difluoro_{"} phenylpyridinate) iridium(III),
bis(4',6'-difluorophenylpyridinato) (3,5-bis(trifluoromethyl)-2-(2'-pyridyl)pyrrolate) iridium(III),
bis(4',6'-difluorophenylpyridinato) (3-(trifluoromethyl)-5-(2-pyridyl)-1,2,4-triazolate) iridium (III),
(Z)-6-mesityl-N-(6-mesitylquinolin-2(1H)-ylidene)quinoline-2-amine-BF₂,
(E)-2-(2-(4-(dimethylamino)styryl)-6-methyl-4H-pyran-4-ylidene)malononitrile, 4-(dicyanomethylene)-2-methyl-6-julolidyl-9-enyl-4-H-pyran, 4-(dicyanomethylene)-2-methyl-6-(1,1,7,7-tetramethyljulolidyl-9-enyl)-4H-pyran,
4-(dicyanomethylene)-2-t-butyl-6-(1,1,7,7-tetramethyl-julolidin-4-ylvinyl)-4H-pyran,
tris(dibenzoylmethane)phenanthroline europium(III), 5,6,11,12-tetraphenylnaphthacene,
bis(2-benzo[b]thiophen-2-yl-pyridine)(acetylacetonate) iridium(III),
tris(1-phenylisoguinoline) iridium(III),
bis(1-phenylisoquinoline)(acetylacetonate) iridium(III), bis[1-(9,9-dimethyl-9H-fluoren-2-yl)isoquinoline] (acetylacetonate) iridium(III),
bis[2-(9,9-dimethyl-9H-fluoren-2-yl)quinoline](acetylacetonate) iridium(III),
tris[4,4'-di-t-butyl-(2,2')-bipyridine]
ruthenium(III)·bis(hexafluorophosphate), tris(2-phenylquinoline) iridium(III),
bis(2-phenylquinoline)(acetylacetonate) iridium(III), 2,8-di-t-butyl-5,11-bis(4-t-butylphenyl)-6,12-diphenyl-tetracene,
bis(2-phenylbenzothiazolate)(acetylacetonate) iridium(III), platinum 5,10,15,20-tetraphenyltetrabenzoporphyrin, osmium(II) bis(3-trifluoromethyl-5-(2-pyridine)pyrazolate)-dimethylphenylphosphine,
osmium(II) bis(3-trifluoromethyl)-5-(4-t-butylpyridyl)-1,2,4-triazolate)diphenylmethylphosphine,
osmium(II) bis(3-(trifluoromethyl)-5-(2-pyridyl)-1,2,4-triazole)dimethylphenylphosphine,
osmium(II) bis(3-(trifluoromethyl)-5-(4-t-butylpyridyl)-1,2,4-triazolate)dimethylphenylphosphine,
bis[2-(4-n-hexylphenyl)quinoline] (acetylacetonate) iridium(III),
tris[2-(4-n-hexylphenyl)quinoline] iridium(III), tris[2-phenyl-4-methylquinoline] iridium(III), bis(2-phenylquinoline)(2-(3-methylphenyl)pyridinate) iridium(III),
bis(2-(9,9-diethylfluoren-2-yl)-1-phenyl-1H-benzo[d]-imidazolato)(acetylacetonate) iridium(III),
bis(2-phenylpyridine) (3-(pyridin-2-yl)-2H-chromen-9-onate) iridium(III),
bis(2-phenylquinoline) (2,2,6,6-tetramethylheptane-3,5-dionate) iridium(III),
bis(phenylisoquinoline) (2,2,6,6-tetramethylheptane-3,5-dionate) iridium(III),
iridium(III) bis(4-phenylthieno[3,2-c]pyridinato-N,C²)acetylacetonate, (E)-2-(2-t-butyl-6-(2-(2,6,6-trimethyl-2,4,5,6-tetrahydro-1H-pyrrolo[3,2,1-ij]quinolin-8-yl)vinyl)-4H-pyran-4-ylidene)-malononitrile,
bis(3-trifluoromethyl-5-(1-isoquinolyl)pyrazolate) (methyldiphenylphosphine) ruthenium,
bis[(4-n-hexylphenyl)isoquinoline](acetylacetonate) iridium(III), platinum(II) octaethylporphin, bis(2-methyldibenzo[f,h]quinoxaline) (acetylacetonate) iridium(III) and
tris[(4-n-hexylphenyl)isoquinoline] iridium(III).

Specific examples of electron transport layer-forming materials include lithium 8-hydroxyquinolinate, 2,2',2"-(1,3,5-benzinetriyl)-tris(1-phenyl-1-H-benzimidazole), 2-(4-biphenyl)-5-(4-t-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine,
3-(4-biphenyl)-4-phenyl-5-t-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene,
1,3-bis[2-(4-t-butylphenyl)-1,3,4-oxadiazo-5-yl]benzene, tris(2,4,6-trimethyl-3-(pyridin-3-yl)phenyl)borane, 1-methyl-2-(4-(naphthalen-2-yl)phenyl)-1H-imidazo-[4,5f][1,10]phenanthroline, 2-(naphthalen-2-yl)-4,7-diphenyl-l,10-phenanthroline, phenyldipyrenylphosphine oxide, 3,3',5,5'-tetra[(m-pyridyl)phen-3-yl]biphenyl, 1,3,5-tris[(3-pyridyl)phen-3-yl]benzene, 4,4'-bis(4,6-diphenyl-1,3,5-triazin-2-yl)biphenyl, 1,3-bis[3,5-di(pyridin-3-yl)phenyl]benzene, bis(10-hydroxybenzo[h]quinolinato)beryllium, diphenylbis(4-(pyridin-3-yl)phenyl)silane and 3,5-di(pyren-1-yl)pyridine.

Examples of electron injection layer-forming materials include lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, lithium acetylacetonate Li(acac), lithium acetate and lithium benzoate.

Examples of cathode materials include aluminum, magnesium-silver alloys, aluminum-lithium alloys, lithium, sodium, potassium and cesium.

In cases where the thin-film obtained from the charge-transporting varnish of the invention is a hole injection layer, another example of a method for fabricating the organic EL device of the invention is as follows.

An organic EL device having a charge-transporting thin-film formed using the charge-transporting varnish of the invention can be produced by, in the fabrication of an EL device as described above, successively forming a hole-transporting and an emissive layer instead of carrying out vacuum evaporation operations for a hole-transporting layer, an emissive layer, an electron-transporting layer and an electron injection layer. Specifically, the charge-transporting varnish of the invention is applied onto an anode substrate, and a hole injection layer is formed by the above-described method. A hole-transporting layer and an emissive layer are then successively formed thereon, following which a cathode material is vapor-deposited on top, thereby giving an organic EL device.

The cathode and anode materials used here may be similar to those described above, and similar cleaning treatment and surface treatment may be carried out.

The method of forming the hole-transporting layer and the emissive layer is exemplified by a film-forming method that entails adding a solvent to a hole-transporting polymer material or a light-emitting polymer material, or to a material obtained by adding a dopant to these, thereby dissolving or uniformly dispersing the material, and then applying the resulting solution or dispersion onto, respectively, the hole injection layer or the hole-transporting layer and subsequently baking the applied layer.

Examples of hole-transporting polymer materials include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis(p-butylphenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)benzidine] end-capped with polysilsesquioxane and poly[(9,9-dioctylfluorenyl-2,7-diyl)-co-(4,4'-(N-(p-butylphenyl))diphenylamine)].

Examples of light-emitting polymer materials include polyfluorene derivatives such as poly(9,9-dialkylfluorene) (PDAF), poly(phenylene vinylene) derivatives such as poly(2-methoxy-5-(2'-ethylhexoxy)-1,4-phenylene vinylene) (MEH-PPV), polythiophene derivatives such as poly(3-alkylthiophene) (PAT), and polyvinylcarbazole (PVCz).

Examples of the solvent include toluene, xylene and chloroform. Examples of the method of dissolution or uniform dispersion include stirring, stirring under applied heat, and ultrasonic dispersion.

Examples of the coating method include, but are not particularly limited to, inkjet printing, spraying, dipping, spin coating, transfer printing, roll coating and brush coating. Coating is preferably carried out in an inert gas atmosphere such as nitrogen or argon.

Examples of the baking method include methods that involve heating in an oven or on a hot plate, either within an inert gas atmosphere or in a vacuum.

An example is described below of a method of fabricating the organic EL device of the invention in a case where the thin-film obtained from the charge-transporting varnish of the invention is a hole injecting and transporting layer.

A hole injecting and transporting layer is formed on an anode substrate, and an emissive layer, an electron-transporting layer, an electron injection layer and a cathode are provided in this order on the hole injecting and transporting layer. Methods of forming the emissive layer, electron-transporting layer and electron injection layer, and specific examples of each, are exemplified in the same way as above.

The anode material, the materials which form the emissive layer, the light-emitting dopant, the electron-transporting layer and the electron-blocking layer, and the cathode material are exemplified in the same way as above.

A hole-blocking layer, an electron-blocking layer or the like may be optionally provided between the electrodes and any of the above layers. By way of illustration, an example of a material that forms an electron-blocking layer is tris(phenylpyrazole)iridium.

The materials which make up the layers that form the anode, the cathode and the layers formed therebetween differ according to whether a device provided with a bottom emission structure or a top emission structure is to be fabricated, and so are suitably selected while taking this into account.

Generally, in an element having a bottom emission structure, a transparent anode is used on the substrate side and light is extracted from the substrate side, whereas in an element having a top emission structure, a reflective anode made of metal is used and light is extracted from a transparent electrode (cathode) in the opposite direction from the substrate. Hence, with regard to the anode material, for example, when fabricating a device having a bottom emission structure, a transparent anode of ITO or the like is used, and when manufacturing a device having a top emission structure, a reflective anode of Al/Nd or the like is used.

The organic EL device of the invention, in order to prevent a deterioration in characteristics, may be sealed in the usual manner with, if necessary, a desiccant or the like.

### EXAMPLES

Working Examples are given below to more concretely illustrate the invention, although the invention is not limited by these Examples. The equipment used was as follows.
(1) ¹H-NMR Measurement:
   Varian high-resolution NMR spectrometer
(2) Substrate Cleaning:
   Substrate cleaning machine (reduced-pressure plasma system), from Choshu Industry Co., Ltd.
(3) Varnish Coating:
   MS-A100 Spin Coater, from Mikasa Co., Ltd.
(4) Film Thickness Measurement:
   Surfcorder ET-4000 microfigure measuring instrument, from Kosaka Laboratory, Ltd.
(5) EL Device Fabrication:
   C-E2L1G1-N Multifunction Vapor Deposition System, from Choshu Industry Co., Ltd.
(6) Measurement of EL Device Brightness:
   I-V-L Measurement System from Tech World, Inc.
(7) Film Surface Examination:
   1LM21D Real Time Scanning Laser Microscope, from Lasertec Corporation

### [1] Compound Synthesis

### [Working Example 1-1]

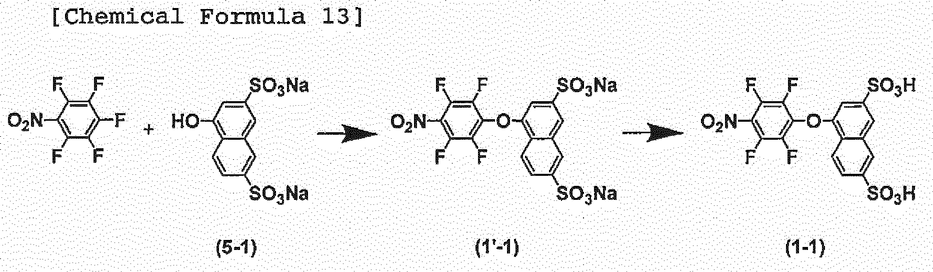

A 100 mL flask was charged with 5.0 g of 1-naphthol-3,6-disulfonic acid disodium salt hydrate, 3.36 g of pentafluoronitrobenzene, 2.18 g of potassium carbonate and 50.0 g of N,N-dimethylformamide and the flask was flushed with nitrogen, following which the mixture was stirred under heating for 1 hour at 100°C.

After the completion of stirring, the reaction mixture was allowed to cool. The solvent was distilled off under reduced pressure from the reaction mixture that had cooled down, the resulting residue was mixed together with 20 mL of methanol, and this mixture was filtered. The filtrate thus obtained was slowly added dropwise to 500 g of isopropanol maintained in a stirred state, following which stirring was carried out for another 30 minutes.

After the completion of stirring, the resulting suspension was filtered, the solvent was removed under reduced pressure from the filtered matter, and the resulting residue thus obtained was dissolved in 20 g of water. Column chromatography was then carried out on the resulting solution using the cation-exchange resin Dowex 650C (about 200 mL of H-type; distillation solvent: water).

Finally, the solvent was distilled off under reduced pressure and the resulting solid was thoroughly dried under reduced pressure, giving Arylsulfonic Acid Compound A (formula (1-1) in a yield of 5.7 g). The ¹H-NMR results were as follows.
¹H-NMR (400 MHz, DMSO-d6) δ [ppm]:
8.31 (s, 1H), 8.26 (d, J = 8.8 Hz, 1H), 8.10 (S, 1H), 7.93 (dd, J = 8.8, 1.6 Hz, 1H), 7.28 (s, 1H).

### [2] Preparation of Charge-Transporting Varnish

### [Working Example 2-1]

The following were dissolved in 8 g of 1,3-dimethyl-2-imidazolidinone (DMI): 0.151 g of Aniline Derivative A of the formula below that was synthesized in accordance with the method described in Bulletin of Chemical Society of Japan, 67, pp. 1749-1752 (1994), and 0.339 g of Arylsulfonic Acid Compound A. To this solution were added 12 g of cyclohexanol (CHA) and 4 g of propylene glycol (PG) and stirring was carried out, giving a charge-transporting varnish.

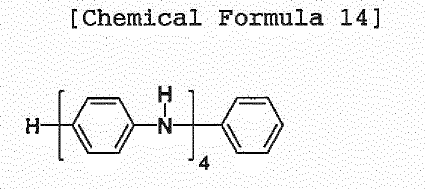

### [Comparative Example 2-1]

The following were dissolved in 8 g of DMI: 0.146 g of Aniline Derivative A and 0.344 g of Arylsulfonic Acid Compound B of the following formula that was synthesized in accordance with the method described in WO 2009/096352. To this were added 12 g of CHA and 4 g of PG and stirring was carried out, giving a charge-transporting varnish.

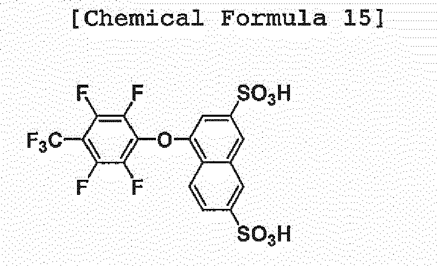

### [3] Production of Charge-Transporting Thin-Film

### [Working Example 3-1]

The charge-transporting varnish obtained in Working Example 2-1 was coated onto an ITO substrate using a spin coater, then pre-baked for 5 minutes at 50°C in open air, and subsequently subjected to a main bake for 15 minutes at 230°C, thereby forming a 30 nm thin-film on an ITO substrate.

A glass substrate with dimensions of 25 mm × 25 mm × 0.7 mm (t) and having indium-tin oxide (ITO) patterned on the surface to a film thickness of 150 nm was used as the ITO substrate. Prior to use, impurities on the surface were removed with an O₂ plasma cleaning system (150 W, 30 seconds).

### [Comparative Example 3-1]

Aside from using the charge-transporting varnish obtained in Comparative Example 2-1 instead of the charge-transporting varnish obtained in Working Example 2-1, thin-film formation was attempted by the same method as in Working Example 3-1.

However, during pre-bake, the liquid film (applied film) ended up agglomerating, as a result of which a thin-film sufficiently uniform for use as the hole injection layer in an organic EL device could not be obtained.

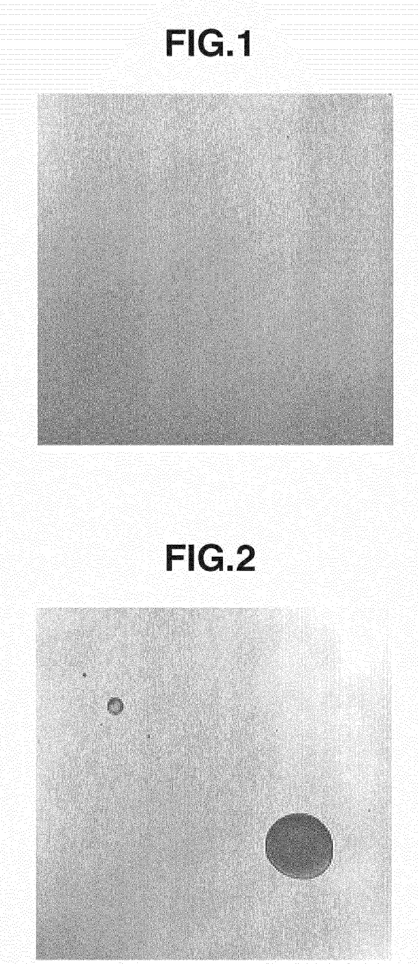

The thin-films obtained in Working Example 3-1 and Comparative Example 3-1 were each examined using a confocal laser microscope. The results are shown in, respectively, FIGS. 1 and 2.

As is apparent from FIGS. 1 and 2, when the varnish of the Comparative Example was used, a thin-film having a high flatness was not obtained; however, when the varnish according to the invention was used, a thin-film having a high flatness was obtained. This is because, by using the varnish of the invention, the liquid film (applied film) did not agglomerate during pre-bake, thus enabling a uniform liquid film state to be maintained.

### [4] Fabrication of Organic EL Devices and Evaluation of Their Characteristics

### [Working Example 4-1]

A uniform 30 nm thin-film was formed on an ITO substrate by the same method as in Working Example 3-1.

Next, using a vapor deposition system (degree of vacuum, 1.0×10⁻⁵ Pa), thin-films of N,N'-di(1-naphthyl)-N,N'-diphenyl-benzidine (α-NPD), tris(8-quinolinolate)aluminum(III) (Alq₃), lithium fluoride and aluminum were successively deposited on the ITO substrate on which a thin-film had been formed, thereby giving an organic EL device. At this time, vapor deposition was carried out at a rate of 0.2 nm/s for α-NPD, Alq₃ and aluminum, and at a rate of 0.02 nm/s for lithium fluoride. The film thicknesses were set to, respectively, 30 nm, 40 nm, 0.5 nm and 120 nm.

To prevent the device characteristics from deteriorating due to the influence of oxygen, moisture and the like in air, the organic EL device was sealed with sealing substrates, following which the characteristics were evaluated. Sealing was carried out by the following procedure.

The organic EL device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the organic EL device, within the sealing substrates.

The laminated sealing substrates were irradiated with UV light (wavelength, 365 nm; dosage, 6,000 mJ/cm²), then annealed at 80°C for 1 hour to cure the adhesive.

The current density, brightness and current efficiency of this device were measured at a driving voltage of 5 V. The results are shown in Table 1.

**[Table 1]**

| | Current density (mA/cm²) | Brightness (cd/m²) | Current efficiency (cd/A) |
|---|---|---|---|
| Working Example 4-1 | 112.5 | 3,150 | 2.8 |

As shown in Table 1, by using the charge-transporting thin-film of the invention as a hole injection layer, an organic EL device having excellent brightness characteristics was obtained.

## Claims

1. An arylsulfonic acid compound of formula (1)
[Chemical Formula 1]
Ar¹-O-Ar² (1)
(wherein Ar¹ is a group of formula (2), and Ar² is a group of formula (3) 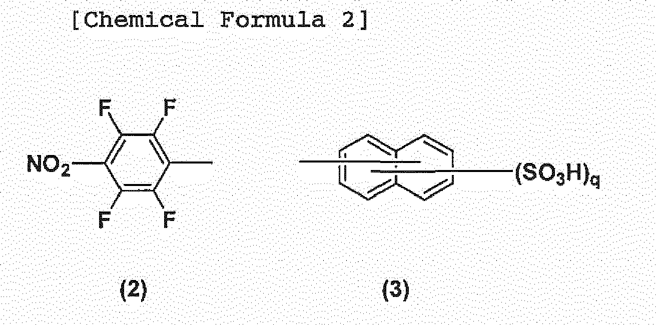 (q being an integer from 1 to 4)).

2. The arylsulfonic acid compound of claim 1 which has any one of formulas (1-1) to (1-4) 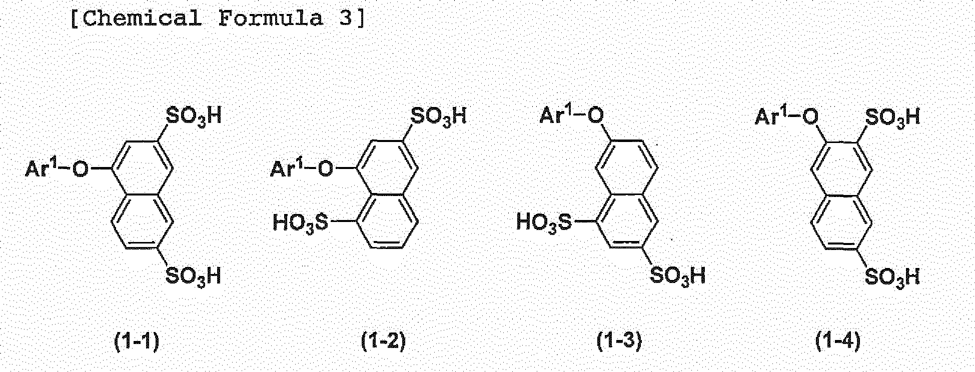 (wherein Ar¹ is as defined above).

3. A dopant consisting of the arylsulfonic acid compound of claim 1 or 2.

4. A charge-transporting material comprising the dopant of claim 3 and a charge-transporting substance.

5. A charge-transporting varnish comprising the dopant of claim 3, a charge-transporting substance and an organic solvent.

6. A charge-transporting thin-film obtained using the charge-transporting varnish of claim 5.

7. An organic electroluminescent device comprising the charge-transporting thin-film of claim 6.

8. A method for producing a charge-transporting thin-film, which method comprises using the charge-transporting varnish of claim 5.

9. A method for preparing the arylsulfonic acid compound of claim 1, which method is **characterized by** comprising the steps of: reacting 2,3,4,5,6-pentafluoronitrobenzene with a naphthalenesulfonic acid salt of formula (5) to form an arylsulfonic acid salt of formula (1') 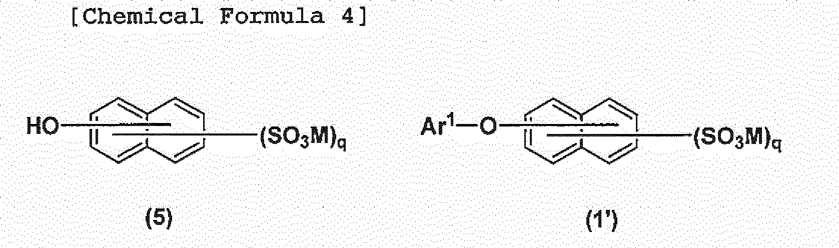 (wherein M is an alkali metal atom, and q and Ar¹ are as defined above); and ion-exchange treating the salt.

## Patentansprüche

1. Arylsulfonsäureverbindung der Formel (1)
[chemische Formel 1]
**Ar¹-O-Ar²** **(1)**
(worin Ar¹ eine Gruppe der Formel (2) ist und Ar² eine Gruppe der Formel (3) ist 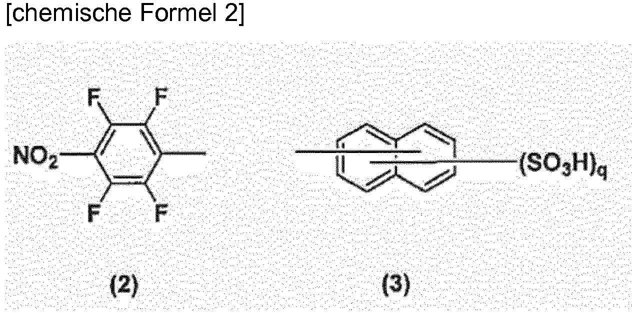 (worin q eine ganze Zahl von 1 bis 4 ist)).

2. Arylsulfonsäureverbindung nach Anspruch 1, die eine der Formeln (1-1) bis (1-4) aufweist 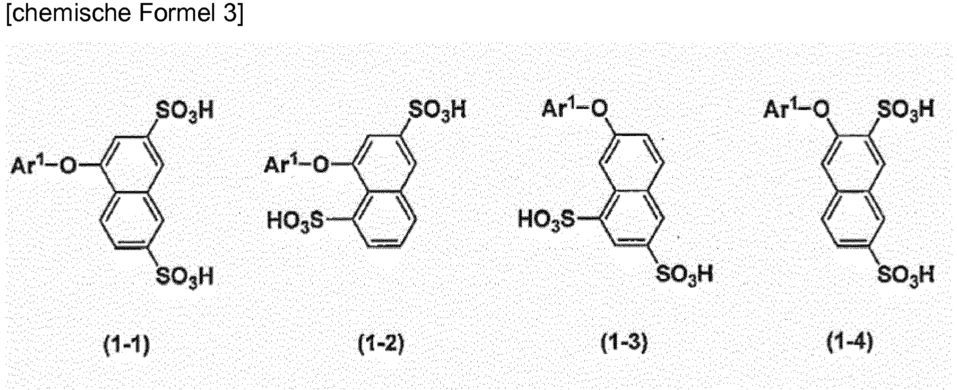 (worin Ar¹ wie oben definiert ist).

3. Dotiermittel, das aus einer Arylsulfonsäureverbindung nach Anspruch 1 oder 2 besteht.

4. Ladungstransportmaterial, das ein Dotiermittel nach Anspruch 3 und eine Ladungstransportsubstanz umfasst.

5. Ladungstransportlack, der ein Dotiermittel nach Anspruch 3, eine Ladungstransportsubstanz und eine organisches Lösungsmittel umfasst.

6. Ladungstransportdünnfilm, erhalten unter Verwendung eines Ladungstransportlacks nach Anspruch 5.

7. Organische elektrolumineszierende Vorrichtung, die einen Ladungstransportdünnfilm nach Anspruch 6 umfasst.

8. Verfahren zur Herstellung eines Ladungstransportdünnfilms, wobei das Verfahren die Verwendung eines Ladungstransportlacks nach Anspruch 5 umfasst.

9. Verfahren zur Herstellung einer Arylsulfonsäureverbindung nach Anspruch 1, wobei das Verfahren **dadurch gekennzeichnet ist, dass** es die folgenden Schritte umfasst: Umsetzen von 2,3,4,5,6-Pentafluornitrobenzol mit einem Naphthalinsulfonsäuresalz der Formel (5), um ein Arylsulfonsäuresalz der Formel (1') zu bilden 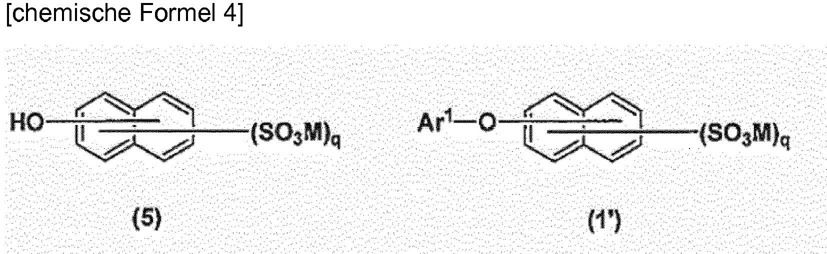 (worin M ein Alkalimetallatom ist und q und Ar¹ wie oben definiert sind); und lonenaustauschbehandeln des Salzes.

## Revendications

1. Composé acide arylsulfonique de formule (1)
[Formule chimique 1]
Ar¹-O-Ar² (1)
(dans laquelle Ar¹ est un groupe de formule (2), et Ar² est un groupe de formule (3) 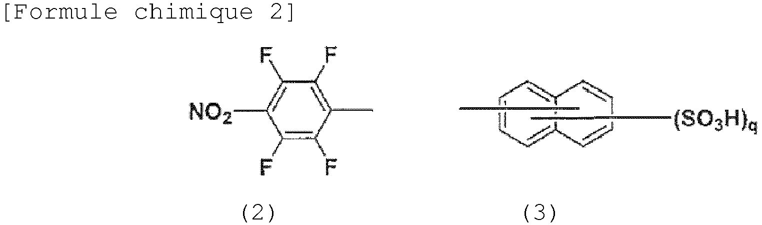 (où q est un entier de 1 à 4)).

2. Composé acide arylsulfonique selon la revendication 1, qui répond à l'une quelconque des formules (1-1) à (1-4) 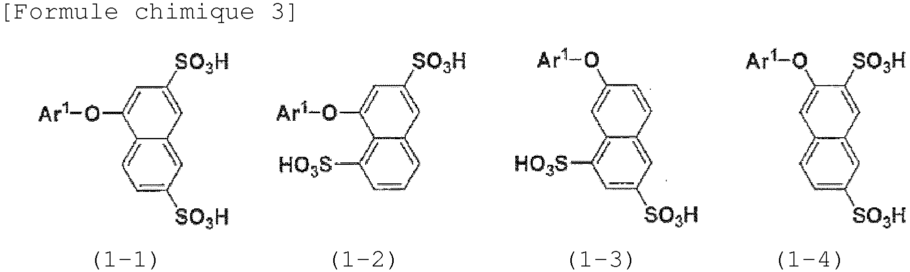 (où Ar¹ est tel que défini ci-dessus).

3. Dopant consistant en le composé acide arylsulfonique de la revendication 1 ou 2.

4. Matériau de transport de charge comprenant le dopant de la revendication 3 et une substance de transport de charge.

5. Vernis de transport de charge comprenant le dopant de la revendication 3, une substance de transport de charge et un solvant organique.

6. Film mince de transport de charge obtenu par utilisation du vernis de transport de charge de la revendication 5.

7. Dispositif électroluminescent organique comprenant le film mince de transport de charge de la revendication 6.

8. Procédé pour produire un film mince de transport de charge, lequel procédé comprend l'utilisation du vernis de transport de charge de la revendication 5.

9. Procédé pour préparer le composé arylsulfonique de la revendication 1, lequel procédé est **caractérisé en ce qu'**il comprend les étapes de : réaction de 2,3,4,5,6-pentafluoronitrobenzène avec un sel d'acide naphtalènesulfonique de formule (5) pour former un sel d'acide sulfonique de formule (1') 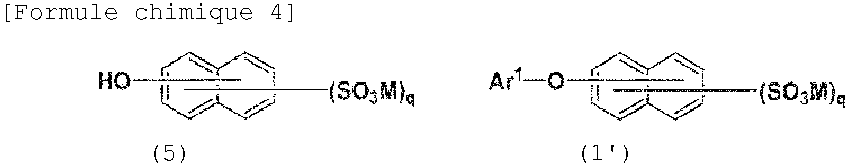 (où M est un atome de métal alcalin, et q et Ar¹ sont tels que définis ci-dessus) ; et de traitement du sel par échange d'ions.
